(19) Europäisches Patentamt
European Patent Office
Office européen des brevets

(11) Publication number: **0 222 155**
**A1**

(12) **EUROPEAN PATENT APPLICATION**

(21) Application number: 86113888.1

(22) Date of filing: 07.10.86

(51) Int. Cl.[4]: **C07D 239/54**, A61K 31/505

(30) Priority: 11.10.85 JP 226609/85

(43) Date of publication of application: 20.05.87 Bulletin 87/21

(84) Designated Contracting States: BE CH DE FR GB IT LI NL SE

(71) Applicant: TERUMO KABUSHIKI KAISHA trading as TERUMO CORPORATION
44-1, 2-chome, Hatagaya Shibuya-Ku
Tokyo 151(JP)

(72) Inventor: Ozawa, Shinji Terumo K.K.
Honmachiryo
25-2, 5-chome, Honmachi Shibuya-ku
Tokyo(JP)
Inventor: Wakabayashi, Toshio
30-30-2, 5-chome, Nagayama
Tama-shi Tokyo(JP)
Inventor: Ozaki, Shoichiro
60-397, Otsu, 1-chome, Takahamacho
Matsuyama-shi Ehime-ken(JP)
Inventor: Katayama, Hajime
6495-1, Igarashi-ichinocho
Niigata-shi Niigata-ken(JP)

(74) Representative: Henkel, Feiler, Hänzel & Partner
Möhlstrasse 37
D-8000 München 80(DE)

# (54) 5-Fluorouracil derivatives.

(57) Novel 5-fluorouracil derivatives are disclosed. As representative examples are mentioned 1-(α-9,12,15-octadecatrienoyloxy-α-methyl)methyl-5-fluorouracil, 1-(α-9,12,15-octadecatrienoyloxy-α-ethyl)methyl-5-fluorouracil, 1-(α-5,8,11,14,17-eicosapentanoyloxy-α-methyl)methyl-5-fluorouracil and the like. These compounds possess potent anticancer activities as well as platelet aggregation-inhibitory activities and are of lower toxicities.

EP 0 222 155 A1

# 5-Fluorouracil derivatives

## Background of the Invention

### 1. Field of the Invention

The present invention relates to novel 5-fluorouracil derivatives. The 5-fluorouracil derivatives provided by the invention are novel compounds which possess potent anticancer activities. The 5-fluorouracil derivatives of the invention also exert potent platelet aggregation-inhibitory activities. Therefore, they are useful for preventing diseases caused by platelet aggregation, namely, thrombosis and the like. Also, as platelet aggregation is known to participate in the metastasis of cancers, the compounds of the invention may be effective in preventing the metastasis of cancers.

### 2. Description of Prior Arts

5-Fluorouracil (5-FU) is clinically applicable as an anticancer agent, however, it is associated with problems of high texicities and narrow ranges of the safe blood level.

Thromboses such as myocardial infarction and cerebral thrombosis have recently become major elements of adult diseases, and development of drugs useful for the prevention of such diseases is highly desirable.

## Summary of the Invention

As a result of extensive studies on the preparation of higher fatty acid esters of 5-fluorouracil and their pharmacological activities, we have found that 5-fluorouracil derivatives according to the invention possess both potent anticancer and platelet aggregation-inhibitory activities. The present invention is based upon the above finding.

It is therefore an object of this invention to provide novel 5-fluorouracil derivatives and anticancer agents and platelet-aggregation inhibitors containing the same. The 5-fluorouracil derivatives according to the invention possess potent anticancer activities. They also have potent platelet aggregation-inhibitory activities and are useful as drugs to prevent diseases caused by the platelet aggregation such as thrombosis and metastasis of cancers.

The present invention comprises 5-fluorouracil derivatives represented by the general formula (I)

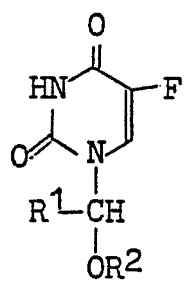

(I)

wherein $R^1$ is an alkyl group containing 1-3 carbon atoms, and $R^2$ is an acyl group derived from a trienoic higher fatty acid or a pentaenoic higher fatty acid.

The invention also comprises anticancer agents containing 5-fluorouracil derivatives represented by the above general formula (I).

Furthermore, the invention comprises platelet aggregation-inhibitory agents containing 5-fluorouracil derivatives represented by the above general formula (I).

As the above-mentioned acyl group derived from a trienoic higher fatty acid is preferred the acyl group derived from α-linolenic acid or dihomo-γ-linolenic acid.

As the above-mentioned acyl group derived from a pentaenoic higher fatty acid is preferred the acyl group derived from eicosapentaenoic acid.

## Detailed Description of the Invention

The above-mentioned 5-fluorouracil derivatives represented by the formula (I) are produced by subjecting 5-FU to condensation reaction with a compound represented by the general formula $Cl\overset{R^1}{\underset{}{C}}H-OR^2$ wherein $R^1$ and $R^2$ are as defined above for the general formula (I) in the presence of a base. Preferably, the reaction is performed under an inert gas such as argon using an organic solvent such as pyridine.

As the base are preferably used tertiary amines such as triethylamine, tributylamine and dimethylaminopyridine.

The 5-fluorouracil derivatives of the invention demonstrate in animal tests antitumor activities equal to or higher than those of 5-FU which is clinically used, and are less toxic than 5-FU. Unlike 5-FU, the 5-fluorouracil derivatives of the invention are characterized by potent platelet aggregation-inhibitory activities.

The 5-fluorouracil derivatives of the invention are usable as the active ingredient or one of the active ingredients in anticancer agents or platelet aggregation-inhibitory agents. As for platelet aggregation inhibition, they are effective for any disease which is caused by platelet aggregation, particularly as an antithrombotic agent or a preventive agent for cardic angina or metastasis of cancers.

The 5-fluorouracil derivatives of the invention may be administered either orally in the form of capsules, tablets, granules or syrup prepared by a conventional method or parentherally in the form of injectable preparations or suppositories.

As examples of the carrier or the excipient are mentioned calcium carbonate, calcium phosphate, starch, sucrose, lactose, talc, magnesium stearate and the like, In addition to the solid preparations, the compounds of the invention may also be formulated into liquid preparations such as oily suspension or syrup.

The compounds of the invention may also be stabilized by inclusion with cyclodextrin.

Platelet aggregation-inhibitory agents containing the 5-fluorouracil derivatives of the invention can be used also for the prevention of blood coagulation in drawing the blood.

Examples and test examples will be given below to describe the invention in more details. The invention, however, is not to be limited thereto.

### Example 1

To 2.22 g of 5-fluorouracil in 20 ml of dry pyridine with stirring in an atmosphere of argon were added 5.82 g of α-chloroethyl 9,12,15-octadecatrienoate and 2.38 ml of triethylamine successively. The mixture was then reacted at 80°C for 5 hours, followed by removal of the pyridine by distillation under reduced pressure. To the residue was added diluted hydrochloric acid, and the mixture was extracted with three portions of methylene chloride. The organic layer of the extract was washed with water and dried over anhydrous magnesium sulfate, and the solvent was removed by distillation under reduced pressure. The extraction residue thus obtained was subjected to column chromatography on silica gel. There was produced 3.50 g of 1-(α-9,12,15-octadecatrienoyloxy-α-methyl)-methyl-5-fluorouracil. Physicochemical data of the product are as follows:

$^1$H-NMR δ (ppm): 0.98(3H, t, J=7Hz), 1.58(3H, d, J=5Hz), 2.02(4H, q, J=5Hz), 2.35(2H, t, J=6Hz), 2.78(4H, t, 4Hz), 6.90(1H, q, J=5Hz), 7.32(1H, d, J=6Hz),
IR $\nu_{max}^{CHCl_3}$ (cm$^{-1}$): 3380, 1755, 1730(Sholder), 1715
MS m/e: 434 (Molecular ion peak), 304, 277, 157

### Example 2

To a mixture of 30 mg of zinc chloride added to 3.60 g of α-linolenoyl chloride at 0°C in an atmosphere of argon was added dropwise 1.16 ml of propionaldehyde with stirring over a period of 15 min. The mixture was reacted at room temperature for 30 min. Water was added to the reaction mixture, which was then extracted with three portions of ether. The organic layer of the extract was washed with water and dried over anhydrous magnesium sulfate. Removal of the solvent by distillation under reduced pressure afforded 4.71 g of α-chloropropyl 9,12,15-octadecatrienoate.

The compound was subjected to reaction with 5-fluorouracil in the same way as in Example 1 to produce 2.72 g of 1-(α-9,12,15-octadecatrienoyloxy-α-ethyl)methyl-5-fluorouracil. Physicochemical data of the product are as follows:

$^1$H-NMR δ (ppm): 7.25(1H, d, J=6Hz)

IR $\nu_{max}^{CHCl_3}$(cm$^{-1}$): 3380, 1755, 1730(Sholder), 1715

MS m/e: 448(Molecular ion peak), 318, 277, 171

Example 3

From 3.91 g of 5,8,11,14,17-eicosapentanoyl chloride and acetaldehyde was prepared, in the same way as in Example 2, 4.86 g of α-chloroethyl 5,8,11,14,17-eicosapentaenoate, which was subjected to reaction with 5-fluorouracil in the same way as in Example 1. There was produced 2.88 g of 1-(α-5,8,11,14,17-eicosapentaenoyloxy-α-methyl)methyl-5-fluorouracil. Physicochemical data of the product are as follows:

IR $\nu_{max}^{CHCl_3}$(cm$^{-1}$): 3380, 1755, 1730(Sholder), 1715

MS m/e: 458(molecular ion peak), 328, 301, 157

Test Examples

1. Antitumor activity

CDF$_1$ mice (male, 5 weeks old) were intraperitoneally transplanted with P388 cells at a dose of 1 x 10$^6$ cells/mouse.

From the next day of the transplantation, a test compound of the invention in the form of 0.5% carboxymethylcellulose suspension was intraperitoneally administered once a day for consecutive five days. Percent increase in life survival was determined:

$$\text{Percent increase in life survival(\% ILS)} = \frac{\text{Central value of survival days for treated group}}{\text{Central value of survival days for control group}} \times 100 - 100$$

Results of the test as compared with 5-fluorouracil are shown in Table 1.

Table 1

| Structural formula | Example No. | $R^1$ | $R^2$ | ILS (%) Dose (mg/kg/day) | | | |
|---|---|---|---|---|---|---|---|
| | | | | 10 | 30 | 40 | 100 |
| 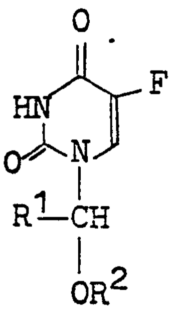 | 1 | $CH_3$ | 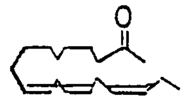 | 24.6 | 46.8 | | 80.5 |
| | 2 | $C_2H_5$ | 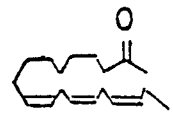 | 27.2 | 39.7 | | 73.8 |
| | 3 | $CH_3$ | 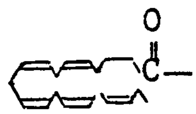 | 26.8 | 49.2 | | 82.1 |
| 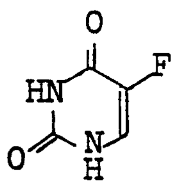 (5-FU) | For comparison | - | - | 65.2 | 27.4 | -24.8 | - |

As clearly seen from Table 1, all the representative compounds of the invention exert antitumor activities equal or better than the activity of 5-FU. Whereas the mice with 5-FU at a dose of 100 mg/kg/day have survival days shorter than those for control mice without 5-FU administration, ILS(%) with the compounds according to the invention shown in Table 1 is 73.8% or higher even at a dose of 100 mg/kg/day, indicating their toxicities lower than those of 5-FU. It has also been demonstrated that compounds of the invention other than those shown in Table 1 possess similar antitumor activities.

2. Platelet aggregation-inhibitory activity

From rabbit carotid vein was drawn blood (9 volumes) by means of a syringe containing 3.8% aqueous sodium citrate (1 volume). The blood was centrifuged to obtain platelet-rich plasma (PRP: 550,000/µl). Platelet aggregation-inhibitory activity of a compound of the invention was measure using as an aggregation-inducing agent arachidonic acid or collagen. 50%-inhibition concentration ($IC_{50}$) against the platelet aggregation caused by arachidonic acid (80 µM) and collagen (30 µg/ml) is shown in Table 2 for representative compounds according to the invention. It is demonstrated that all of the compounds of the invention tested possess potent platelet aggregation-inhibitory activities as compared with 5-FU. It has also been confirmed that 5-fluorouracil derivatives according to the invention not shown in Table 2 possess similar antitumor activities.

3. Acute toxicity

An acute toxicity test was run with ICR male mice (7 weeks old) by an oral administration. As compared with 5-fluorouracil with an $LD_{50}$ of 309 mg/kg, $LD_{50}$ values with all the compounds of the invention were 800 mg or higher thereby demonstrating their higher safety.

Table 2

| Structural formula | Example No. | $R^1$ | $R^2$ | 50%-Inhibition concentration(mole) Arachidonic acid | Collagen |
|---|---|---|---|---|---|
| | 1 | $CH_3$ | 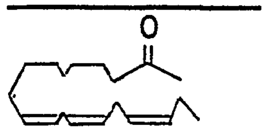 | $6.5 \times 10^{-5}$ | $3.4 \times 10^{-4}$ |
| 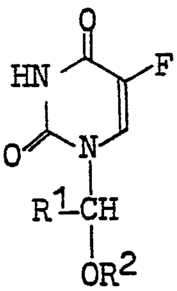 | 2 | $C_2H_5$ | 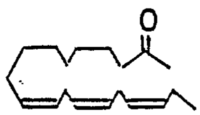 | $5.7 \times 10^{-5}$ | $5.3 \times 10^{-4}$ |
| | 3 | $CH_3$ | 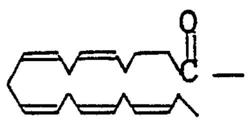 | $3.2 \times 10^{-5}$ | $1.4 \times 10^{-4}$ |
| 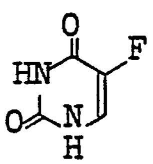 (5-FU) | For comparison | - | - | $>10^{-3}$ | $>10^{-3}$ |

**Claims**

1. A 5-fluorouracil derivative represented by the general formula (I)

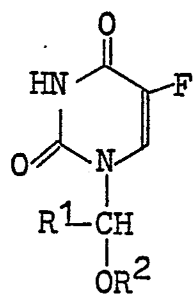

(I)

wherein $R^1$ is an alkyl group containing 1-3 carbon atoms and $R^2$ is an acyl group derived from a trienoic higher fatty acid or a pentaenoic higher fatty acid.

2. A 5-fluorouracil derivative according to Claim 1 wherein the acyl group derived from a trienoic higher fatty acid is an acyl group derived from α-linolenic acid or dihomo-γ-linolenic acid.

3. A 5-fluorouracil derivative according to Claim 1 wherein the acyl group derived from a pentaenoic higher fatty acid is an acyl group derived from eicosapentaenoic acid.

4. An anticancer agent containing a 5-fluorouracil derivative represented by the general formula (I) of Claim 1.

5. An anticancer agent according to Claim 4 wherein the acyl group derived from a trienic higher fatty acid is an acyl group derived from α-linolenic acid or dihomo-γ-linolenic acid.

6. An anticancer agent according to Claim 4 wherein the acyl group derived from a pentaenic higher fatty acid is an acyl group derived from eicosapentaenoic acid.

7. A method for treating cancer which comprises administering a therapeutically effective amount of the 5-fluorouracil derivative according to Claim 1 to a mammal possibly afflicted with said disease.

8. A platelet aggregation-inhibitory agent containing a 5-fluorouracil derivative represented by the general formula (I) of Claim 1.

9. A platelet aggregation-inhibitory agent according to Claim 8 wherein the acyl group derived from a trienic higher fatty acid is an acyl group derived from α-linolenic acid or dihomo-γ-linolenic acid.

10. A platelet aggregation-inhibitory agent according to Claim 8 wherein the acyl group derived from a pentaenic higher fatty acid is an acyl group derived from eicosapentaenic acid.

11. A method for preventing a disease caused by aggregation of the platelet which comprises administering a therapeutically effective amount of the 5-fluorouracil derivative according to Claim 1 to a mammal possibly afflicted with said disease.

12. A method of preventing a disease of Claim 11 wherein the disease is thrombosis.

European Patent Office

# PARTIAL EUROPEAN SEARCH REPORT

which under Rule 45 of the European Patent Convention shall be considered, for the purposes of subsequent proceedings, as the European search report

Application number

EP 86113888.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A,P | EP - A1 - 0 162 924 (TERUMO)<br>* Claims 1-5,8-12,15-19 *<br>& WO-A1-8501729 (25.04.1985)<br>-- | 1,2,4,<br>5,8-10 | C 07 D 239/54<br>A 61 K 31/505 |
| A | EP - A1 - 0 046 307 (CHUGAI SEIYAKU)<br>* Claim 1, abstract *<br>---- | 1,4-6,<br>8-10 | |

TECHNICAL FIELDS SEARCHED (Int. Cl.4)

C 07 D 239/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6,8-10

Claims searched incompletely:

Claims not searched: 7,11,12

Reason for the limitation of the search:

(Article 52(4) EPC; method for treatment of human or animal body by therapy)

| Place of search | Date of completion of the search | Examiner |
|---|---|---|
| VIENNA | 16-01-1987 | LUX |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1505.1. 03.82